(19) Europäisches Patentamt / European Patent Office / Office européen des brevets

(11) **EP 4 130 294 A1**

(12) **EUROPEAN PATENT APPLICATION**
published in accordance with Art. 153(4) EPC

(43) Date of publication:
**08.02.2023 Bulletin 2023/06**

(21) Application number: **21774585.0**

(22) Date of filing: **24.03.2021**

(51) International Patent Classification (IPC):
***C12Q 1/6851*** (2018.01)

(86) International application number:
**PCT/CN2021/082743**

(87) International publication number:
**WO 2021/190566 (30.09.2021 Gazette 2021/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.03.2020 CN 202010229992**

(71) Applicant: **Flashdx Shenzen Inc.
Shenzhen, Guangdong 518057 (CN)**

(72) Inventor: **YUE, Min
Shenzhen, Guangdong 518057 (CN)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

Remarks:
The complete document including Reference
Table(s) and the Sequence Listing(s) can be
downloaded from the EPO website

(54) **UNIVERSAL PROBE CHIP-BASED MULTIPLEX QUANTITATIVE PCR TESTING SYSTEM**

(57) Provided is a surface probe-based quantitative PCR testing system, comprising: (a) a solid phase carrier; (b) a specific primer pair of a sequence to be tested, which comprises a first primer and a second primer; and (c) a quenching probe. Further provided are a method for quantitative PCR testing and a kit.

EP 4 130 294 A1

## Description

### Technical field

The present invention relates to the field of nucleic acid detection, and more specifically to a universal probe chip-based multiplexed quantitative PCR detection system.

### Background

In conventional Taqman fluorescent quantitative PCR (Taqman qPCR), primer pairs for amplification and Taqman probes for detection are present in the detection system. Wherein the Taqman probe in solution has a fluorescent group (fluorophore) on one side (or one end) and a quenching group (quencher) on the other side (or the other end). When the probe is intact, because the quencher and the fluorescent agent are close enough to each other, the quencher can effectively inhibit the fluorescent agent so that there is no (or essentially no) fluorescent signal. When a nucleic acid sample matching the sequence of this probe is present in the solution, then during the process of the sample being amplified by PCR, the probe is cut by the amplification enzyme, the fluorescent agent and the quencher will then be detached from each other and, resulting in reduced or absent inhibition, thus allowing the fluorescent agent to produce a fluorescent signal.

In the case of multiplex amplification for amplification, different probes and primers can easily cause interference. An effective approach, especially in the case of relatively high multiple amplification number, an effective approach is to use nested PCR but nested PCR generally requires a two-step approach to do, with the addition of primers and enzymes in between, as well as dilution of non-specific products.

For multiplex qPCR, because each sequence to be tested requires a fluorescent agent whose luminescence spectrum can be distinguished from other fluorescent agents, the number of different nucleic acids that can be detected simultaneously in the same qPCR reaction is greatly limited, and usually only 4-5 multiplexes can be achieved in a single qPCR reaction. In addition, even if multiplex qPCR can be achieved, the corresponding optical detection equipment is complex in design and very expensive because of the need to read several different fluorescent agents at the same time. Optimization requires extensive experiments and probe synthesis is also expensive.

There is therefore an urgent need in the field to develop a universal microarray-based multiplex PCR primer design and amplification system for the purpose of economically quantifying and detecting multiplex specific sequences.

### Summary of the invention

It is an object of the present invention to provide a universal microarray-based multiplex PCR primer design and amplification system for the purpose of economically quantifying multiplex specific sequences.

In a first aspect of the present invention, it provides a surface probe-based quantitative PCR detection system, the detection system comprising.

(a) a solid support, the solid support having n sub-detection zones on a main surface, wherein n is a positive integer of & 2 and at least one of the sub-detection zones is a surface quantification sub-detection zone; wherein each of the surface quantification sub-detection zone is independently immobilized with a microarray surface probe, the microarray surface probe being a single stranded nucleic acid, and one end of the microarray surface probe is immobilized on the surface of the solid support, and the microarray surface probe carries a first detectable marker, the first detectable marker being selected from the group consisting of: a fluorescent group, luminescent group, luminescent marker, quantum dot, and a combination thereof;
(b) a primer pair specific to the sequence to be detected, comprising a first primer and a second primer;
(c) a quenching probe, a quencher is attached to one side or one end or in the middle of the quenching probe;

and wherein the quenching probe and at least one microarray surface probes of the surface quantification sub-detection zone can be combined to form a double-stranded structure, and in the double-stranded structure, the quenching group of the quenching probe causes the signal of the first detectable marker (e.g. fluorescent group) of the microarray surface probe to be quenched in whole or in part; when the concentration of the quenching probe in the detection system is reduced, the degree of quenching of the signal of the first detectable marker (e.g. fluorescent group) of the microarray surface probe of at least one surface quantification sub-detection zone is reduced.

In another preferred embodiment, the first primer is a forward specific primer.

In another preferred embodiment, the second primer is a reverse specific primer.

In another preferred embodiment, the detection system further comprises: (d) a universal primer pair comprising a forward universal primer and a reverse universal primer (i.e. a fourth primer and a fifth primer).

In another preferred embodiment, the quenched in part means the degree of quenching of the signal of the first detectable marker (e.g. fluorescent group) of the microarray surface probe after addition by & 50%, preferably, & 70%, more preferably & 80%, compared to that before addition of the quenching probe.

In another preferred embodiment, the reduced degree of quenching means that as the concentration of the quenching probe in the detection system decreases, the degree of quenching of the signal of the first detectable marker (e.g. fluorescent group) of the microarray surface probe is ≤ 50%, preferably ≤ 30%, more preferably, ≤ 20%.

In another preferred embodiment, the first primer has a structure of Formula I:

$$5'\text{-}T_{ai}'\text{-}L_{ai}\text{-}C_a\text{-}L_{bi}\text{-}P_i\text{-}L_{ci}\text{-}T_{bi}\text{-}T_{ai}\text{-}3'\ (\text{I})$$

wherein $i$ is the $i$ number of a multiplex (n') detection, $i$ is a positive integer and $1 \le i \le n'$; n' is a positive integer and $1 \le n' \le$ the number of a solid support sub-detection zone, preferably n' is 2-100, more preferably, n' is 3-20;

$T_{ai}$ is the a-part of the forward-specific sequence of the $i$-th target gene.

$T_{ai}'$ is a reverse complementary sequence of $T_{ai}$.

$T_{bi}$ is the b-part of the forward specific sequence of the $i$-th target gene; and $T_{ai}$, $T_{bi}$ are directly adjacent to each other and together form the forward specific sequence of the target gene;

$P_i$ is the sequence of the marker probe (barcode index probe) that marks the amplification of the $i$ number of gene;

$C_a$ is the sequence of the forward universal amplification primer;

$L_{ai}$, $L_{bi}$, $L_{ci}$ each independently being none or a flexible transition region, the flexible transition region being selected from the group consisting of: a flexible transition nucleic acid fragment of 1-15 nt in length, a flexible transition polymer fragment of 1-10 nt in length, and a combination thereof;

each "-" is independently a bond or a nucleotide linker sequence.

In another preferred example, $L_{ai}$, $L_{bi}$, $L_{ci}$ may be the same and may be different.

In another preferred example, when i=1, the first primer has a structure as shown in Formula V:

$$5'\text{-}T_{a1}'\text{-}L_{a1}\text{-}C_a\text{-}L_{b1}\text{-}P_1\text{-}L_{c1}\text{-}T_{b1}\text{-}T_{a1}\text{-}3'\ (\text{V})\ ;$$

wherein

$T_{a1}$ is the a-part of the forward-specific sequence of the 1st target gene.

$T_{a1}'$ is the reverse complementary sequence of $T_{a1}$.

$T_{b1}$ is the b part of the forward-specific sequence of the 1st target gene; and $T_{a1}$, $T_{b1}$ are directly adjacent to each other and together form the forward-specific sequence of the target gene;

$P_1$ is the sequence of the marker probe (barcode index probe) that marks the amplification of the 1st number of gene;

$C_a$ is the sequence of the forward universal amplification primer;

$L_{a1}$, $L_{b1}$, $L_{c1}$ each independently being none or a flexible transition region, the flexible transition region being selected from the group consisting of: a flexible transition nucleic acid fragment of 1-15 nt in length, a flexible transition polymer fragment of 1-10 nt in length, and a combination thereof;

each "-" is independently a bond or a nucleotide linker sequence.

In another preferred example, $T_{ai}$ has a length of 6-20bp, preferably, 8-16bp, more preferably, 9-12bp.

In another preferred example, the length of $T_{bi}$ is 3-50bp, preferably, 5-22bp, more preferably, 10-15bp.

In another preferred example, $P_i$ has a length of 10-200bp, preferably, 15-100bp, more preferably, 20-30bp.

In another preferred example, $C_a$ has a length of 15-50bp, preferably, 18-40bp, more preferably, 20-35bp.

In another preferred example, $T_{ai}'$ has a length of 6-20bp, preferably, 8-16bp, more preferably, 9-12bp.

In another preferred example, the second primer has a structure of Formula II:

$$5'\text{-}C_b\text{-}L_{di}\text{-}T_{revi}'\text{-}3'\ (\text{II})$$

wherein

$C_b$ is a reverse universal amplification primer sequence;

$L_{di}$ is none or a flexible transition region, the flexible transition region being a flexible transition nucleic acid fragment of 1-10 nt in length;

$T_{revi}'$ is a specific sequence at the 3' end of the *i* number targeting gene.

In another preferred example, the length of $C_b$ is 15-50bp, preferably, 18-40bp, more preferably, 20-35bp.

In another preferred example, $T_{revi}'$ has a length of 15-70bp, preferably, 18-38bp, more preferably, 20-35bp.

In another preferred example, the quenching probe has a structure of Formula III:

$$5'\text{-}Q\text{-}P_i\text{-}3' \quad (\text{III})$$

wherein Q is a quenching group;

$P_i$ is a sequence of the marker probe (barcode index probe) that marks the amplification of the *i* number of gene.

In another preferred example, $P_i$ is 10-200 bp in length, preferably 15-100 bp, more preferably, 20-30 bp.

In another preferred example, the microarray surface probe has a structure of Formula IV:

$$5'\text{-}D\text{-}L_{ei}\text{-}S_i\text{-}L_{fi}\text{-}3' \qquad (\text{IV})$$

wherein

D is a linker group to which the microarray surface probe is attached to the solid support;

$L_{ei}$ is none or a flexible transition region, the flexible transition region being selected from the group consisting of: a flexible transition nucleic acid fragment of 1-100 nt in length, a flexible transition polymer fragment of 1-10 nt in length, and a combination thereof;

$S_i$ is a capture region, wherein the sequence of the capture region is substantially or fully complementary to the sequence of $P_i$;

$L_{fi}$ is none or a 3' end sequence region of 1-20 nt in length;

i is a positive integer in the interval from 1 to n' and $1 \leq n' \leq$ the number of the solid support sub-detection zone, preferably n' is 2-100, more preferably, n' is 3-20.

In another preferred example, a first detectable marker (e.g. a fluorescent group) is attached to one end or one side or in the middle of the microarray surface probe.

In another preferred example, the first detectable signal is located at $S_i$ or $L_{fi}$, preferably at $S_i$.

In another preferred example, $L_{fi}$ is none.

In another preferred embodiment, on the solid support, the number of the surface quantification sub-detection zone is m, m being a positive integer of & 1 and $m \leq n$.

In another preferred embodiment, n is 2-500, preferably 5-250, more preferably 9-100 or 16-96.

In another preferred embodiment, m is 2-500, preferably 5-250, more preferably 9-100 or 16-96.

In another preferred embodiment, m = n.

In another preferred embodiment, the same first detectable marker is used in at least & 2 (preferably & 3) of the surface quantification sub-detection zone.

In another preferred embodiment, each surface quantification sub-detection zone is used to detect different or identical target sequences.

In another preferred embodiment, each surface quantification sub-detection zone is used to detect a different target sequence.

In another preferred embodiment, said first detectable marker is a fluorescent group.

In another preferred embodiment, the fluorescent group is selected from the group consisting of: a luminescent label.

In another preferred embodiment, the fluorescent group is selected from the group consisting of quantum dots.

In another preferred embodiment, the signal of the first detectable marker (e.g. fluorescent group) of the microarray surface probe is fully or partially quenched when the ratio (Q1/Q0) of the number Q1 of the quenching probe within effective hybridization distance of the microarray surface probe to the number Q0 of the corresponding microarray surface probe is 2-100 , preferably 5-50.

In another preferred embodiment, when the quenching probe is degraded or partially degraded such that the ratio (Q1/Q0) of the number Q1 within effective hybridization distance to the microarray surface probe to the number Q0 of the corresponding microarray surface probe is reduced to 0-10, preferably 0-2, the quenching degree of the signal of the first detectable marker (e.g. fluorescent group) of the microarray surface probe is significantly reduced, the detectable signal of the surface probe is significantly enhanced.

In another preferred embodiment, the detection system further comprises one or more components selected from the group consisting of:

(e) a buffer or buffer composition for PCR amplification;
(f) a polymerase for PCR amplification;
(g) a positive universal amplification primer $C_a$;
(h) a negative universal amplification primer $C_b$.

In another preferred example, the polymerase is selected from the group consisting of: Taq enzyme, Pfu enzyme, Pwo enzyme, vent enzyme, KOD enzyme, superfi enzyme, and a combination thereof.

In another preferred example, the length (bp) of the amplification product amplified by the first primer and the second primer is 50-2000, preferably 75 -300, more preferably, 75-150.

In another preferred example, the microarray surface probe for the different surface quantification sub-detection zone is a same or different nucleic acid molecule.

In another preferred embodiment, the first primer, the second primer, and/or quenching probe comprises DNA, RNA, and a combination thereof.

In another preferred embodiment, the first primer has a length (nt) of 35-150, preferably 60-120, more preferably, 70-100.

In another preferred embodiment, the second primer has a length (nt) of 35-150, preferably 55-100, more preferably, 70-80.

In another preferred embodiment, the quenching probe has a length (nt) of 12-50, preferably 15-40, more preferably, 20-30.

In another preferred embodiment, the microarray surface probe has a length (nt) of $\leq$ 150, preferably $\leq$ 60, more preferably $\leq$ 40.

In another preferred embodiment, the microarray surface probe has a capture region that is substantially or fully complementary to the quenching probe.

In a second aspect of the invention, it provides a method for performing a quantitative PCR detection comprising the steps of:

(a) providing a sample to be tested and a quantitative PCR detection system based on a surface probe as described in the first aspect of the present invention;
(b) performing PCR amplification of the sample to be tested with the quantitative PCR detection system under suitable conditions for PCR amplification.
(c) detecting the signal of a first detectable marker of one or more surface quantification sub-detection zones on a solid support during or after the PCR amplification; and
(d) analyzing the detected signal of the first detectable marker, thereby obtaining a quantitative detection result of the sample to be tested.

In another preferred embodiment, in step (d), the analysis comprises comparing the detected signal of the first detectable marker with a standard curve.

In another preferred embodiment, in step (d), the analysis comprises comparing the detected signal of the first detectable marker with the detection signal before amplification.

In another preferred embodiment, the quantitative PCR detection is a multiplex PCR detection.

In another preferred embodiment, the first detectable marker is the same for each surface quantification sub-detection zone.

In another preferred embodiment, the first detectable marker is different for each surface quantification sub-detection zone.

In a third aspect of the invention, it provides a kit for a quantitative PCR detection comprising:

(a) a first container and a solid support located in the first container ,

the solid support having n sub-detection zones on a main surface, wherein n is a positive integer of & 2 and at least one of the sub-detection zones is a surface quantification sub-detection zone;
wherein each of the surface quantification sub-detection zone is independently immobilized with a microarray surface probe, the microarray surface probe being a single stranded nucleic acid, and one end of the microarray surface probe is immobilized on the surface of the solid support, and the microarray surface probe carries a first detectable marker, the first detectable marker being selected from the group consisting of: a fluorescent group, luminescent group, luminescent marker, quantum dot, and a combination thereof;

(b) a second container and (b1) a primer pair specific to the sequence to be detected, comprising a first primer, a second primer; and (b2) a quenching probe, a quencher is attached to one side or one end or in the middle of the quenching probe, located in the second container;

and wherein the quenching probe and at least one microarray surface probes of the surface quantification sub-detection zone can be combined to form a double-stranded structure, and in the double-stranded structure, the quenching group of the quenching probe causes the signal of the first detectable marker (e.g. fluorescent group) of the microarray surface probe to be quenched in whole or in part; when the concentration of the quenching probe in the detection system is reduced, the degree of quenching of the signal of the first detectable marker (e.g. fluorescent group) of the microarray surface probe of at least one surface quantification sub-detection zone is reduced;

(c) optionally a third container and a buffer or buffer component for PCR amplification located in the third container;

(d) optionally a fourth container and a polymerase for PCR amplification located in the fourth container;

(e) optionally a fifth container and a universal amplification primer pair located in the fifth container; and

(f) optionally a specification, the specification describes a method for performing a quantitative PCR detection.

In another preferred example, the first container, second container, third container, fourth container, and fifth container are the same container or different containers.

It should be understood that, within the scope of the present invention, each technical feature of the present invention described above and in the following (as examples) may be combined with each other to form a new or preferred technical solution, which is not listed here due to space limitations.

## Description of Figure

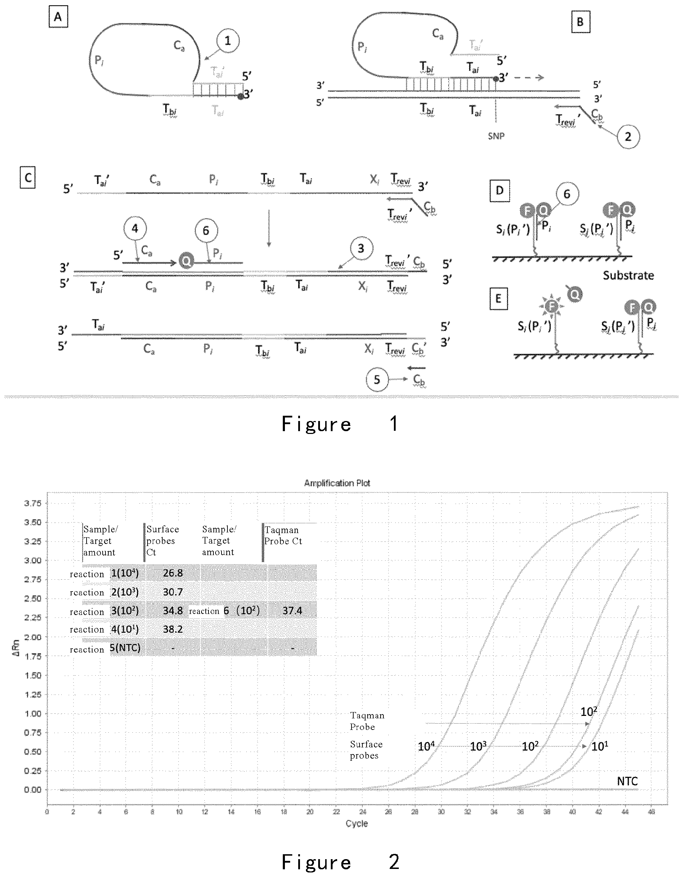

Figure 1 shows the design principle and schematic implementation of multiplex PCR amplification. (A) The structure of the first primer (forward specific primer) in the $i$ number reactive system of multiplex reaction is designed as 5'-$T_{ai}$'-$L_{ai}$-$C_a$-$L_{bi}$-$P_i$-$L_{ci}$-$T_{bi}$-$T_{ai}$-3'. $T_{ai}$, $T_{bi}$ is a linked forward specific sequence for targeting genes, equivalent to a forward amplification primer for normal PCR; $L_{ai}$, $L_{bi}$, $L_{ci}$ is a linked flexible transition region; $C_a$ is a forward universal amplification primer; $P_i$ is s barcode index sequence of marker gene amplification combination; $T_{ai}$' is a reverse complementary sequence of $T_{ai}$. Under normal conditions, $T_{ai}$'-$T_{ai}$ forms a stem-loop structure to stabilize the primers and reduce the reaction-specific background. (B) A diagram of primer binding during amplification. In the presence of the target $T_{bi}$-$T_{ai}$ sequence in the reaction system, the forward-specific primer binds to its negative strand because ($T_{bi}$-$T_{ai}$) binding to its complementary strand with greater stability than $T_{ai}$'-$T_{ai}$, and the forward-specific primer provides the primer-target binding required for PCR extension. Optionally, the SNP detection site or specific differentiation site can be designed at the 3' end of $T_{ai}$. Reverse specific primers are designed as overhang primers for normal PCR: $T_{revi}$' specifically binds the targeting sequence and $C_b$ is a reverse universal amplification primer sequence. (C) Schematic diagram of PCR amplification. The quenching probe (6) binds to the amplification product amplified by the specific primer pair, Cleavage of the quenching probe by nucleic acid polymerase during amplification can result in the quencher Q being cleaved away from the quenching probe sequence (the quenching probe is degraded). (D) In the absence of amplification, the quenching probe (6) binds to the microarray surface probe (7) corresponding to its specific sequence, inhibiting the luminescence of the microarray dot matrix on the surface of the chip. (E) In the case of specific amplification, the quencher Q is cleaved away from the quenching probe sequence (the quenching probe is degraded), the probe without the quencher or without the intact quenching probe can bind to the surface probe and an increase in light signal can be detected at the corresponding point on the microarray dot matrix.

Figure 2 shows the results of the technical feasibility validation of the method in the present invention for PCR amplification applications. The results show that the reaction system can achieve good detection of the target to be tested in a feasibility experiment that has not yet been optimized. Reactions 1-5 use a combination of specific primer pairs, universal primer pairs, quenching probes and microarray surface probes (a seventh probe), while reaction 6 uses a combination of primer pairs and Taqman probes from conventional Taqman qPCR for the target gene. The amplification curve is as expected within the $10^1$-$10^4$ copy target input interval (reactions 4, 3, 2, 1). The average Ct per 10-fold target dilution is approximately 3.8. Also the negative control NTC (reaction 5) does not amplify significantly. As a comparison, the conventional Taqman probe (reaction 6) also obtains specific amplification, Ct is slightly lagged when comparing the same $10^2$ copy input target (reaction 3) (37.4 vs 34.8). This experimental result confirms the technical feasibility and advancement of the present invention.

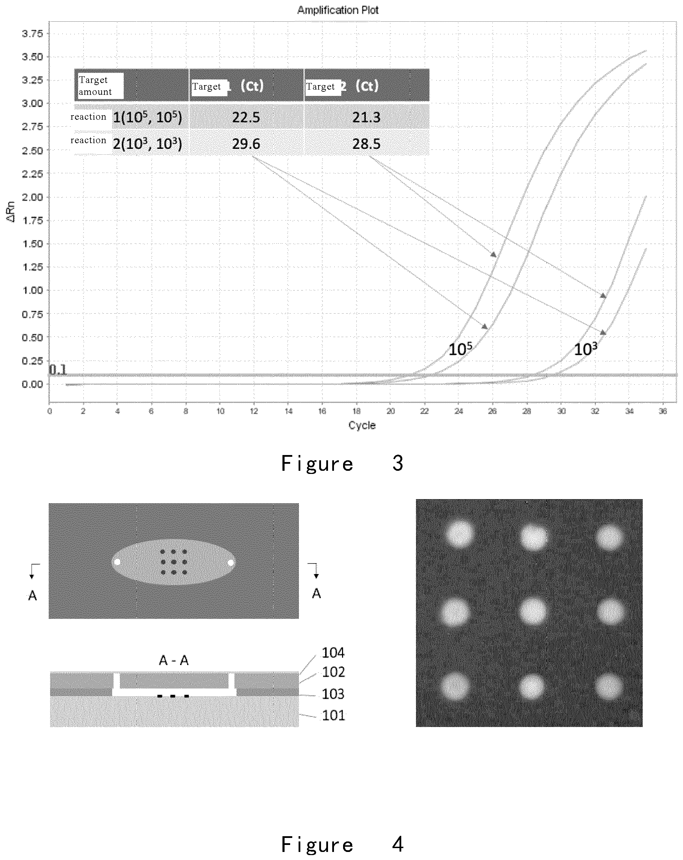

Figure 3 shows the results of the technical feasibility validation of the method in the present invention for multiplex PCR amplification. It can be observed from the experimental results that the double amplification of this reaction system allows independent, specific and quantitative detection of double amplification products at $10^5$ and $10^3$

orders of magnitude copy input in feasibility experiments. In reactions 1 and 2, for the detection of the first of amplification (target 1), Ct = 7.2 and for the detection of the second amplification (target 2), Ct = 7.1 and the Ct of the two detection targets is also relatively close.

Figure 4 shows the detection chip and sub-detection zone. A diagram of the detection chip is shown on the left, and the top left is a top view of the detection chip, below left is a cross-sectional view of the construction of the detection chip. On the right is a photograph of the nine sub-detection zones on the chip.

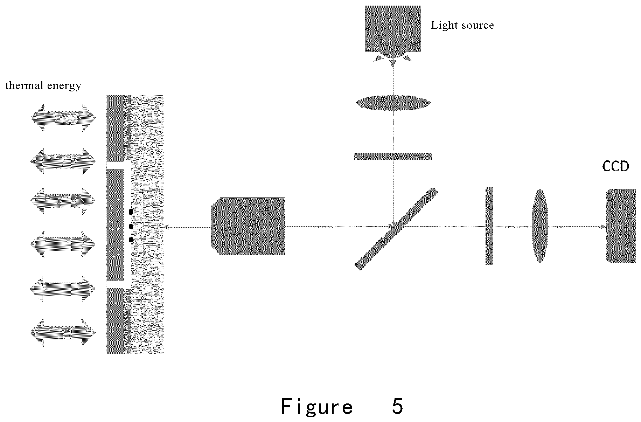

Figure 5 shows a schematic diagram of the light path and temperature control of the detection device.

**Detailed Description**

After extensive and intensive research, the present inventors have developed, for the first time, a highly specific multiplex nucleic acid detection using only a pair of low concentration target-specific primers and a series of quenching probes. While the nucleic acids are amplified, at each amplification cycle, the quenching probes can be read quantitatively by the microarray surface probes immobilized to the surface to achieve quantitative detection for specific sequences. In addition, even if only one or two or a few detectable markers and the corresponding quencher are used, the detection effect of the invention is not affected, thus making both manufacturing and usage costs significantly reduced. The present invention has been completed on this basis.

**Terms**

As used herein, the term "primer" refers to a synthetic short nucleic acid fragment (in particular a DNA fragment) that determines the start and stop positions to be amplified in a polymerase chain reaction.

As used herein, the term "quenching probe" or "quenching probe of the present invention" is used interchangeably to refer to a probe with a quencher.

As used herein, the terms "microarray surface probe", "microarray surface probe of the present invention", "surface probe", "surface probe of the present invention" are used interchangeably and refer to a single-stranded nucleic acid molecule with a first detectable marker (e.g., a fluorescent group) that is used to capture a quenching probe and is immobilized on the surface of a solid support. It should be understood that the microarray surface probes of the present invention are different from probes in the free state (which carry both a fluorescent group and a quencher) of prior art (e.g. fluorescent quantitative PCR).

As used herein, the terms "sub-detection zone" and "array points" are used interchangeably.

**Detection system**

The present invention provides a surface probe-based quantitative PCR detection system, the detection system comprising.

(a) a solid support, the solid support having n sub-detection zones on a main surface, wherein n is a positive integer of & 2 and at least one of the sub-detection zones is a surface quantification sub-detection zone;
wherein each of the surface quantification sub-detection zone is independently immobilized with a microarray surface probe, the microarray surface probe being a single stranded nucleic acid, and one end of the microarray surface probe is immobilized on the surface of the solid support, and the microarray surface probe carries a first detectable marker, the first detectable marker being selected from the group consisting of: a fluorescent group, luminescent group, luminescent marker, quantum dot, and a combination thereof;
(b) a primer pair specific to the sequence to be detected, comprising a first primer and a second primer;
(c) a quenching probe, a quencher is attached to one side or one end or in the middle of the quenching probe;

and wherein the quenching probe and at least one microarray surface probes of the surface quantification sub-detection zone can be combined to form a double-stranded structure, and in the double-stranded structure, the quenching group of the quenching probe causes the signal of the first detectable marker (e.g. fluorescent group) of the microarray surface probe to be quenched in part; when the concentration of the quenching probe in the detection system is reduced, the degree of quenching of the signal of the first detectable marker (e.g. fluorescent group) of the microarray surface probe of at least one surface quantification sub-detection zone is reduced (i.e. the proportion of the signal that is quenched is correspondingly lower).

**Microarray surface probes, amplification primers and quenching probes**

As shown in Figure 1A and 1B, initial amplification is mainly achieved by a pair of target-specific primers, namely the first primer and the second primer.

The first primer is the 5' end primer with the structure $T_{ai}'-L_{ai}-C_a-L_{bi}-P_i-L_c-_iT_{bi}-T_a$ and the second primer is the 3' end primer $C_b-L_{di}-T_{rev\,i}'$. The T in the structure designation refers to the target region (Target) to be detected; L refers to the Linker region; C refers to the Constant primer, also known as the universal primer; $C_a$, $C_b$ are the forward and reverse universal amplification primers; (') refers to the nucleotide antisense complementation; the numerical subscript $i$ ($1 \leq i \leq n'$) refers to the i(n') number of the multiplex detection; L refers to the optional Linker region; wherein $1 \leq n'$ A the number of a solid support sub-detection zone, preferably n' is 2-100, more preferably, n' is 3-20.

The specific sequence at the 3' end of the first primer ($T_{bi}-T_{ai}$) is identical to the specific sequence of the target to be tested (a sequence specific to the sequence to be tested), and its 5' end $T_{ai}'$ is an antisense complementary sequence to $T_{ai}$, in order to sufficiently influence the differentiation of the primer annealing efficiency.

The first primer, because it also contains a hairpin structure, the $T_{ai}$ region in $T_{bi}-T_a$ at the 3' end is complementary to the $T_{ai}'$ region at the 5' end so that before the first primer binds to the substrate to be tested, complementary hybridization of $T_{ai}$ and $T_{ai}'$ regions to form a hairpin structure, capable of partially stabilising the structure (Figure 1A). In the presence of a well paired $T_{bi}-T_{ai}$ region of the target substrate to be tested, $T_{ai}$ and $T_{ai}'$ unwind the hairpin structure as $T_{bi}-T_{ai}$ full region binding provides a more stable structure (Figure 1B). In this case, the $T_{ai}'-C_a-P_i$ region of the first primer forms the 5' end of the overhang and the 3' end of $T_{bi}-T_{ai}$ binds the substrate to be tested to form an effective extension. This strand extension and the second primer are able to form an efficient amplification pair. As shown in Figure 1C, extension of the primer (a first primer) at the 5' end produces the $T_{ai}'-C_a-P_i-T_{bi}-T_a-X-T_{revi}$ sequence, with X being the sequence in the target region between the first and second primers. The corresponding second primer at the 3' end binds this forward strand and extends to produce its complementary strand $C_b-T_{revi}'-X'-T_{ai}'-T_{bi}'-P_i'-C_a'-T_{ai}$ (a third amplification product).

After the third amplification product is generated in the reaction system, a universal primer pair including the forward universal primer $C_a$ (a fourth primer) and the negative universal primer $C_b$ (a fifth primer) can produce efficient amplification for the third amplification product (Figure 1C). This double amplification can be performed in the same reaction system as the initial amplification (a first primer and a second primer) at the same time.

One of the probes for quantitative detection is the quenching probe, i.e. a sixth probe $Q-P_i$, with the quencher Q linked at the 5' end or 3' end or in the middle (preferably the 5' end).

In the multiple reaction design, each number reaction corresponds to a separate set of a first primer, second primer and quenching probe ($Q-P_i$), but shares a fourth primer (forward universal primer), and a fifth primer (reverse universal primer). A series of non-interfering probes ($P_1 ... P_i ...P_n$) to form a marker probe (barcode index probe) series for universal marker multiplex amplification combination. The $P_i$ sequences themselves are independent of their corresponding target sequences to be detected, i.e. they are not correlated with the target sequences to be tested. The combination of marker probes can therefore be a combination of groupings of each of the different marker probes in order to achieve the detection of different sets of targets to be tested with the same set of probes.

When the fourth primer is extended for the amplification of the third amplification product, which corresponds to the degradation of the Taqman probe, the sixth probe is quantitatively degraded. This leads to a reduction in the effective concentration of the quenching probe $Q-P_i$ with the quencher in the amplification system.

There is a seventh probe on the microarray chip, i.e. microarray surface probes series, in which the probe sequence ($S_1 ...S_i...S_n$) and a sixth probe barcode index sequence ($P_1 ...P_i..P_n$) are reverse complement. The sequences of the seventh probe $S_i$ and the sixth probe $P_i$ are reverse complement (Fig. 1D, E). Without amplification, $S_i$ carries the luminescent group F and is bound by an excess of $P_i$ with a quencher that does not emit light or has low luminescence intensity. When quantitative amplification leads to a decrease in the concentration of the corresponding quenching probe (the quenching probe is degraded), the luminophore of $S_i$ produces quantitative fluorescence.

In the present invention, the same pair (or a few pairs, e.g. 2-5 pairs) of universal primers are used for the specific amplification of each target nucleic acid sequence to be detected.

In a preferred embodiment,

$T_{ai}$ length is 6-20bp, preferably, 8-16bp, more preferably, 9-12bp.
$T_{bi}$ length is 3-50bp, preferably 5-22bp, more preferably, 10-15bp.
$T_{revi}$ length is 9-70bp, preferably, 13-38bp, more preferably, 20-35bp.
$C_a$ length is 15-50bp, preferably, 18-40bp, more preferably, 20-35bp.
$P_i$ length is 10-200bp, preferably, 15-100bp, more preferably, 20-30bp.
$X_i$ length is 2-500bp, preferably 15-99bp, more preferably, 20-50bp.
$L_i$ length is 0-20bp, preferably, 0-6bp, more preferably, 0-2bp.

**Solid support**

In the present invention, the surface probe (microarray surface probe) is immobilized to the surface quantification sub-detection zone of the solid support.

Typically, the 5' end of the surface probe (microarray surface probe) is closer to the solid surface, while the 3' end is further away from the solid surface.

**Surface probe quantitative PCR method**

The present invention provides a surface probe quantitative PCR method.

For ease of understanding, the present inventors describe it in conjunction with a schematic diagram (Figure 1). It should be understood that the scope of protection of the present invention is not limited by the principles or schematics as described.

As shown in Figure 1D, in the present invention, instead of using a solution probe with both a fluorescent agent and a quencher, a quenching probe with only a quencher, and a surface probe with a fluorescent agent immobilized to a physical surface are used to achieve quantitative PCR by detecting changes in the fluorescence signal of the fluorescent probe on the physical surface.

Prior to amplification, the quenching probe with quencher in solution hybridizes with the surface probe, so that the probe points on the surface is in the dark state.

If there is a target to be tested that corresponds to the forward and reverse specific primers and forms a primer-substrate effective hybridization, a valid amplification is formed (Figure 1-C). The universal primer performs a effective secondary amplification of the amplification product generated by the specific primer simultaneously, and the effective amplification is accompanied by the specific degradation of the quenching probe containing the barcode index sequence. The quenching probe is degraded or loses its quencher, resulting in the inability to hybridize effectively with the microarray surface probe. The fluorescence signal of the surface probe changes from a dark state where it is inhibited before amplification to a luminescent state where the inhibition decreases during amplification and even finally to a completely uninhibited state (Figure 1E). Real-time quantification of the target to be measured by detecting the real-time fluorescence signal of the surface probe. In the method as described, the presence of the target nucleic acid first generates specific exponential amplification, resulting in enhanced fluorescence of the surface probe, the degree of fluorescence enhancement is directly related to the degree of speed of the amplification reaction, thus allowing quantification of the target nucleic acid, with the signal intensity and speed corresponding to the initial concentration of the target to be measured.

In terms of multiplex detection, since the detection signal is derived from the surface probe, and since the physical location of the surface probe itself can be used to distinguish the corresponding target nucleic acid on it, multiplex detection can be achieved with just one marker, without the need for multiple fluorescent agents of traditional multiplex qPCR. The method can be used to quantify at least 1-100 target nucleic acids, or more than 100. In contrast, traditional multiplex qPCR techniques are limited by the type of fluorescent agents and optical design, and can usually only do 4-6 numbers of real-time quantification detection, with very few being able to do 8 numbers.

In the present invention, however, it is very suitable for multiplex quantification detection, especially multiplex real-time quantification detection. In the present invention, the number of multiplex is not particularly limited and can be any positive integer & 2, preferably 2-10000 or 2-500, preferably 3-250, more preferably 2-100.

Typically, in the present invention, signal readings of the surface probe are performed at selected times and temperatures for each cycle (either every 1 or every 2 cycles) during the amplification reaction.

**Kit**

The present invention also provides a kit for quantitative PCR detection, which comprises:

(a) a first container and a solid support located in the first container ,

the solid support having n sub-detection zones on a main surface, wherein n is a positive integer of & 2 and at least one of the sub-detection zones is a surface quantification sub-detection zone;
wherein each of the surface quantification sub-detection zone is independently immobilized with a microarray surface probe, the microarray surface probe being a single stranded nucleic acid, and one end of the microarray surface probe is immobilized on the surface of the solid support, and the microarray surface probe carries a first detectable marker, the first detectable marker being selected from the group consisting of: a fluorescent group, luminescent group, luminescent marker, quantum dot, and a combination thereof;

(b) a second container and (b1) a primer pair specific to the sequence to be detected, comprising a first primer, a second primer; and (b2) a quenching probe, a quencher is attached to one side or one end or in the middle of the quenching probe, located in the second container;
and wherein the quenching probe and at least one microarray surface probes of the surface quantification sub-detection zone can be combined to form a double-stranded structure, and in the double-stranded structure, the quenching group of the quenching probe causes the signal of the first detectable marker (e.g. fluorescent group) of the microarray surface probe to be quenched in part; when the concentration of the quenching probe in the detection system is reduced, the degree of quenching of the signal of the first detectable marker (e.g. fluorescent group) of the microarray surface probe of at least one surface quantification sub-detection zone is reduced (i.e. the proportion of the signal that is quenched is correspondingly lower);

(c) optionally a third container and a buffer or buffer component for PCR amplification located in the third container;

(d) optionally a fourth container and a polymerase for PCR amplification located in the fourth container;

(e) optionally a fifth container and a universal amplification primer pair located in the fifth container; and

(f) optionally a specification, the specification describes a method for performing a quantitative PCR detection.

In addition, the kit of the present invention may contain other components or parts, such as standard curves, quality control samples, etc.

In the present invention, any two, three or all of the first, second, third, fourth containers, and fifth containers are the same container.

In another preferred embodiment, the first, second, third, fourth and fifth containers are different containers.

In one embodiment, all of the reagents themselves can be present in the first container.

In another preferred embodiment, all reagents (enzymes, primers, salts) are stored in the first container in a lyophilised form and the DNA to be tested is melted in the corresponding buffer and injected into the first container at the time of the reaction.

**Reaction device and detection system**

The present invention also provides a reaction device and a detection system for surface probe-based quantitative PCR.

A representative reaction device is shown in Figure 4.

In the present invention, the chip used for the reaction can take any form, as long as the reaction chamber has an inner surface on which microarrays (arrays of surface quantification sub-detection zone) can be prepared in advance and subsequent reading of the optical signal can be performed.

A typical design is a flat reaction cavity design as shown in Figure 4.

The reaction cavity is shown in Figure 4 and consists of three parts. The first part 101 is a piece of plastic or glass, the second part 102 is another piece of flat plastic or glass bonded by a third part of 103 pieces of 0.25mm thick double-sided adhesive. The double-sided adhesive has a cutting part to form a reaction chamber. The reaction chamber can also consist of only two parts: the first part already contains the grooves that form the reaction chamber, the second part can have a single-sided adhesive that can be directly bonded to the first part, or it can be formed into a closed reaction chamber with the first part by means of ultrasonic fusion, thermal fusion, double-sided adhesive or UV Light Adhesives. The chamber has entry and exit channels or inlet and outlet for the inflow of the reaction solution, and after the reaction solution has been added, the valves on the channels can be closed or the inlet and outlet can be permanently closed. In this example, a 0.1 mm thick single-sided adhesive 104 is used to permanently close the inlet and outlet.

One inner surface of the reaction chamber is surface chemically treated and spot sampled with microarrays (surface quantification sub-detection zone array) prior to reaction chip integration. As an example, Figure 4 shows a 3x3 microarray. The array is used to detect 3 different nucleic acids (2 target nucleic acids and 1 internal reference), corresponding to each nucleic acid sequence to be tested with three sub-detection zones immobilized with surface probes corresponding to the nucleic acid to be tested. The use of more than one sub-detection zone for the detection for each nucleic acid sequence to be tested allows for confirmation of the target nucleic acid to be tested when the detection signal of one of the sub-detection zones is interfered with due to bubbles or impurities and there are still other points of sequence identity. The number of sub-detection zones forming the microarray can be 2-100, or more than 100.

The material on one side of the reaction chamber must have sufficient transmission in the relevant band of the fluorescent agent (excitation and emission), at least 80%, preferably 90% or more, and the lower the autofluorescence of the material on that side, the better. In this example, the first part of the reaction chamber is a low autofluorescence slide (BOROFLOAT® 33 from Schott), the material has a light transmission of over 90% for visible light and much lower autofluorescence than ordinary glass materials.

The surface nucleic acid probe sequence is usually coupled to the solid surface by a small linker.

In the present invention, the linker may be an oligonucleotide (oligo), a polymer (e.g. PEG), or a combination.

The detection signal in the present invention is the fluorescence of the surface probe. The surface probe is dark before amplification and hybridization of the quenching probe to the surface probe results in quenching of the fluorescence of the surface probe due to the FRET reaction. To improve hybridization efficiency, the length of the coupling linker should be no less than 5 nm and preferably more than 10 nm so that the surface probe available for hybridization is far enough away from the solid surface to allow sufficient hybridization with the quenching probe. The solid surface is usually treated with surface chemistry to produce reactive groups such as NHS esters, Thioester etc. that can react with the coupling linker. The linkage between the coupling linker and the solid surface is thermally stable and is not broken or detached by the cooling heating cycle of the PCR. Slides with a surface layer of NHS ester available for coupling are used in this example (such slides are available from ArrayIt and others). The surface probe is a synthetic sequence with an amino group synthesized at the 5' end that can be covalently coupled to the NHS ester on the surface of the slide. The amino group is linked to the DNA sequence by the coupling linker polyethylene glycol chain $[PEG]_{50}$ (polyethylene glycol). The sequence of the DNA is identical to the barcode index $P_i'$ on the second probe of each target to be tested, respectively (i.e. complementary to its corresponding amplification product) and coupled with the fluorescent agent Cy3 at its 3' end.

Surface probe arrays (i.e. sub-detection zone arrays) can be generated using conventional methods of generating microarrays. In this example a contactless spotting machine from Scienion was used to spot the sample. A contact spotting machine such as the SpotBot® from ArrayIt is also a common tool. The diameter of the array of points (i.e. each sub-detection zone) in this example is approximately 50 $\mu$m and the edge-to-edge spacing of each point is approximately 100 $\mu$m.

The total number of each surface probe should be comparable to the corresponding number of intact quenching probes prior to amplification in the solution near that array point, such that the luminophore on the surface probe are quenched as much as possible prior to amplification. When the quenching probe in the solution near the array point is degraded due to amplification, the concentration of intact quenching probes gradually decreases and the array point will gradually have surface probes without quenching probes hybridizing with it and quenching its luminophore, thus the fluorescence signal of the array point gradually increases until finally there are no intact quenching probes hybridizing with it at all and the fluorescence signal of the array point reaches its maximum. The surface probe density at each point on the microarray should be higher than 500 fmole/cm$^2$ and preferably higher than 2000 fmole/cm$^2$. Before amplification, the quenching probe hybridizes with the surface probe and the fluorescent agent on the surface probe is almost completely quenched, resulting in the lowest fluorescence brightness at the array point. When the corresponding target nucleic acid is present and specifically amplified, the quenching probe is degraded, the number of intact quenching probes that can hybridize with their corresponding surface probes gradually decreases, and the number of unquenched surface probe luminescent groups gradually increases, and the fluorescence brightness at the array point reaches the highest value when the quenching probe is completely degraded. During the cycle, as the quenching probe decreases, the surface probe gradually increases its own fluorescence signal due to a reduction in hybridization with a quenching probe that quenches its signal. If the initial target nucleic acid concentration is relatively low, the quenching probe is degraded later and more slowly, and therefore the fluorescence enhancement of the surface probe also occurs later and more slowly. There is a one-to-one and monotonically decreasing relationship between the fluorescence brightness of the array points and the number of quenching probes, so that the degree and speed of fluorescence change at each array point can be used to quantify the concentration of the nucleic acid corresponding to its initial target.

The heating and cooling cycle control for PCR can be performed on one or both sides of the reaction chamber at the same time. Simultaneous temperature control on both sides increases the rate of temperature regulation of the reaction chamber and thus reduces the reaction time. For the purpose of this example, it is preset that the heating and cooling cycle is carried out from one side only. The thinner the reaction chamber, the faster the temperature regulation and the easier it is for the reaction chamber solution to reach thermal equilibrium at each temperature point, regardless of whether temperature control is carried out on one or both sides, so that the thickness of the reaction chamber is usually below 2 mm, preferably below 1 mm, and in this example a design of 0.25 mm is used. The thickness of the solid material on the temperature controlled side ensures the strength of the chip, the thinner the material, the faster the heating and cooling cycle. In this example it is a 0.5mm polycarbonate sheet. Also, because the fluorescence signal is read from one side of the glass, black polycarbonate is used here to further reduce background noise during fluorescence reading.

There are several common methods of heating and cooling cycles for PCR, such as control of metal modules in contact with the reaction chamber with thermoelectric modules, water baths, oil baths or heating the solution directly with infrared light. In this example, a computer-controlled thermoelectric module heats and cools the copper block, which, because of its high thermal conductivity, allows the reaction chamber to reach thermal equilibrium more quickly with each temperature change.

The microarray fluorescence signal reading on the inner surface of the reaction chamber can be done using a common monochrome fluorescence signal reading system. Figure 5 depicts a typical monochromatic fluorescence signal reading system. In this example, the fluorescence signal of the surface probe in the microarray is acquired using a fluorescence microscope (Olympus IX73), the principle of which is consistent with the system depicted in the figure above.

**Applications**

The method of the invention is particularly beneficial for multiplexed quantitative detection of nucleic acids.

Conventional qPCRs that require multiplex detection require a different fluorophore for each marker to be tested, which makes the optical design of the device very complex and has a limited spectrum that allows for limited multiplicity. The present invention transfers the signal to be measured from solution to a physical surface, which can be spot sampled at multiple locations, each of which can be designed with different surface probe sequences and quenching probes for different targets to be measured, but each of which can use the same fluorophore, thus enabling multiplex quantitative detection under one fluorophore. Ideally 2X-200X is supported, but also multiplexed detection of 200-1,000X or 1,000-10,000X can be supported.

In terms of application areas or occasions, the invention can be used for the identification of microorganisms and other fields where multiplex quantitative PCR is applied, such as oncogene detection, genotyping, etc. For example, in the case of upper respiratory tract infections, quantitative detection can be performed by designing multiplex surface probes and quenching probes for specific sequences of common viruses and bacteria to identify the specific microbial source of infection. The invention can also identify subtypes of microorganisms, for example multiplex surface probes can be designed to quantify the specific sequences of different subtypes of HPV, allowing the typing of HPV samples in a single reaction.

**The main advantages of the present invention include.**

(a) Highly specific multiplex amplification using a pair of low concentration target-specific primers and a series of quenching probes, while amplifying, at each amplification cycle, microarray surface probes immobilized on the surface can quantify reads of quenching probes for sequence-specific quantitative detection.
(b) Enables rapid and easy quantitative analysis.
(c) A large number of target sequences can be detected simultaneously in parallel.
(d)Universal probe series for efficient reduction of production costs, shortened product development cycles and interference optimization (one-off optimization, as the probe series is designed to be reused)
(e) Simultaneous amplification with two number primer pairs, with a high concentration of universal primer pairs and a low concentration of specific primer pairs, to achieve high sensitivity while effectively maintaining low interference.
(f) Reduce non-specific amplification by effectively stabilizing the secondary structure. It is also possible to effectively differentiate SNPs and obtain additional SNP typing functions.

The present invention is further described below in connection with specific embodiments. It should be understood that these embodiments are intended to illustrate the invention only and are not intended to limit the scope of the invention. Experimental methods for which specific conditions are not indicated in the following embodiments generally follow conventional conditions, such as those described in Sambrook et al, Molecular Cloning: A Laboratory Manual (New York: Cold Spring Harbor Laboratory Press, 1989), or as recommended by the manufacturer. Unless otherwise stated, percentages and parts are percentages by weight and parts by weight.

Materials and reagents in embodiments are commercially available products if not otherwise stated.

**Example 1 Universal qPCR reaction and detection surface probe-based**

1. Reactants in amplification reactions.

There are six reactions in this example, using amplification substrates in the following order: in reaction cassettes 1-5, different concentrations of target synthetic sequence substrate 1 were added. The substrate copy numbers were approximately $10^4$, $10^3$, $10^2$, 10 and 0 respectively (blank control NTC). The copy number of the target substrate added to cassette 6 is $10^2$, the same as for cassette 3.

2. Methods:

A two-stage, three-step PCR was used. The first stage of amplification is a three-step PCR wherein the fluorescence signal is read either at annealing or at amplification (extension), preferably at amplification. The PCR temperature is set at:

a. Thermal denaturation at 95 °C for 5 minutes

b. 15 thermal cycles, each cycle comprising

    i. 95 °C for 15 seconds
    ii. 65 °C for 30 seconds
    iii. 72 °C for 30 seconds

The second stage of amplification comprises 25 thermal cycles, each cycle comprising:

    i. 95 °C for 15 seconds
    ii. 60 °C for 30 seconds
    iii. 72 °C for 30 seconds

3. Primers, probes

The primers used for amplification were:
a first primer ($T_{a1}'$-$L_a$-$C_a$-$L_b$-$P_1$-$T_{b1}$-$T_{a1}$) (SEQ ID NO.: 1) :

$$\underline{\text{5'-TGTTGCGTTC}}\text{-}\textbf{TC}\text{-}\underline{\text{AATGATACGGCGACCACCGA}}\text{-}\textbf{TT}\text{-}\underline{\text{ACCATGCAG}}$$

$$\underline{\text{AAGGAGGCAAA}}$$

【　　　$T_{a1}'$　　】 $L_{a1}$ 【　　　　　$C_a$　　　　】$L_{b1}$【　　　　$P_1$

$$\underline{\text{GTAAGGAGG}}\text{-}\textbf{TTTGGACTGA-}\underline{\text{GAACGCAACA}}\text{-3'}$$

】【　$T_{b1}$　　】【　$T_{a1}$　　】

The primer example has a total of 83 nucleotides (10+2+20+2+29+10+10=83).
A second primer ($C_b$-$T_{rev1}'$) (SEQ ID NO.:2) :

$$\text{5'-}\underline{\text{CAAGCAGAAGACGGCATACGAGAT}}\text{-}\textbf{ACAGGCTGGCTCAGGACTATCT}\text{-3'}$$

【　　　　$C_b$　　　　】【　　　　$T_{rev1}'$　　　　】

This primer example has a total of 46 nucleotides.
A fourth primer ($C_a$ Universal primer) < SEQ ID NO.:3 ) : 5'-AATGATACGGCGACCACCGA-3' (20 nucleotides)。
A fifth primer ($C_b$ Universal primer) < SEQ ID NO.:4 ) : 5'-CAAGCAGAAGACGGCATACGAGAT-3' (24 nucleotides)。
A sixth probe (Q-$P_1$ probe):
5'-BHQ-ACCATGCAGAAGGAGGCAAAGTAAGGAGG (SEQ ID NO.:5) -3' (29 nucleotides)。BHQ stands for Black Hole Quencher and can be other equivalent fluorescent annihilating groups.
A seventh probe ($S_1$ probe):
5'-F-CCTCCTTACTTTGCCTCCTTCTGCATGGT < SEQ ID NO.:6) -3' (29 nucleotides)。F is a fluorescent group such as FAM, Cy3.
Target substrate 1 (SEQ ID NO.:7) :

5'-<u>TTTGGACTGAGAACGCAACA</u>-**CATGGAGGTTGCTAGTCAGGCCA**

-<u>GGACAAATGGTG</u>

【 $T_{b1}$-$T_{a1}$ 】 【 $X_1$ 5' interval 】

【 Target conventional

<u>CAGGCAATGAGAGAG</u>-**CCATTGGGACTCATCCCAG**-<u>AGATAGTCCTGAGCCAGCCTGT</u>-3'

<u>Taqman probe</u> 】 【 $X_1$ 3' interval 】 【 $T_{rev1}$ 】

Target conventional Taqman probe (partial sequence of $X_1$ target amplicon):
5'-F-GGACAAATGGTGCAGGCAATGAGAGAGAG (SEQ ID NO.:8) -Q-3'
The primer pairs added to the conventional Taqman reaction are:
Forward is 5'- $C_a$-$T_{b1}$-$T_{a1}$-3':

5'-<u>AATGATACGGCGACCACCGA</u>-**TTTGGACTGA**-<u>GAACGCAACA</u>-3'

（SEQ ID NO.:9）

【 $C_a$ 】 【 $T_{b1}$-$T_{a1}$ 】

Reverse primer is the same as the second primer (5'-$C_b$-$T_{rev1}$'-3').
The amplification reagent mixture described for the PPCR amplification reaction contains:

- Standard PCR reagents: 1X Fast Start Taq (0.2 unit/ul), 2 mM MgCl2, 0.5 mg/ml BSA, 1x Fast Start buffer
- A First primer, A second primer (10nM) (reactions 1-6)
- A Fourth primer, A fifth primer (100nM) (reactions 1-6)
- Quenching (a sixth) probe Q- $P_1$ (100nM) (reactions 1-6)
- Traditional amplification primer (100nM) and Taqman probe (100nM) for the target region (reaction 7)

The results of the experiment are shown in Figure 2. It can be observed that this reaction system can achieve a better detection of the detection target in a feasibility experiment that has not yet been optimized. Reactions 1-5 use a reaction combination of specific primer pairs, universal primer pairs, quenching probes (a sixth probe) and microarray surface probes (a seventh probe), while reaction 6 uses a combination of conventional Taqman qPCR reactions for the target gene (containing conventional Taqman reaction primer pairs and Taqman probes). The expected amplification curves and Ct~ concentration relationships were obtained from target input intervals of $10^1$-$10^4$ copies (samples 4, 3, 2, 1). The average Ct per 10-fold input target dilution (from Ct= 26.8 for reaction 1 to 38.2 for reaction 4) is approximately 3.8. After optimization of the reaction conditions (e.g. reaction annealing temperature), the Ct should be improved considerably. Meanwhile the negative control NTC does not amplify significantly. For comparison, the conventional Taqman probe for the target gene in the same system (sample 6) is also amplified specifically, with a slight lag in Ct (37.4 vs 34.8) when comparing the same $10^2$ copies of the input target (sample 3). This experiment validates the technical feasibility and advancement of the system.

**Example 2: System validation of multiplex PCR**

This embodiment further validates that the invention can support two or more effective amplifications. Each is equivalent to the system validated in Example 1, independently quantified and amplified and detected by the microarray chip. The target substrate for the first amplification, each primer pair and probe is the same as in Example 1, including the first, second, fourth and fifth primers, sixth and seventh probes, and target substrate 1. The concentrations are also equivalent to Example 1.
The second amplification uses target substrate 2 and its corresponding first primer, second primer, sixth probe and seventh probe, as described below. The fourth primer and fifth primer (universal primer pair) are shared by multiplex reactions and do not need to be added again. There are two reactions in this embodiment, in reaction A, both target substrate copy numbers are approximately $10^5$ and in reaction B, both target substrate copy numbers are approximately $10^3$.

Reaction conditions are the same as in Example 1.

Primers, probes

The primers used for the second amplification were:

A first primer ($T_{a2}'-L_a-C_a-L_b-P_2-T_{b2}-T_{a2}$) ( SEQ ID NO.:10) :

5'-<u>CGATGCCAGT</u>-**TC**-<u>AATGATACGGCGACCACCGA</u>-**TT**-<u>CCCTCAACG</u>

<u>GTATCGCGTC</u>

【   $T_{a2}'$   】$L_{a2}$【      $C_a$      】$L_{b2}$【      $P_2$

<u>GGTTGC</u>- **TGTGTACTAC**- <u>ACTGGCATCG</u>-3'

  】【   $T_{b2}$   】【   $T_{a2}$   】

The primer example has a total of 83 nucleotides (10+2+20+2+29+10+10=83).

A second primer ($C_b-T_{rev2}'$) (SEQ ID NO.: 11) :

5'-<u>CAAGCAGAAGACGGCATACGAGAT</u>- GTACTCAGGCTGACAGTAAGTCTTAG-3'

   【      $C_b$      】【    $T_{rev2}'$     】

This primer example has a total of 46 nucleotides.

Quenching (a sixth) probe (Q- $P_2$ probe):

5'-BHQ-CCCTCAACGGTATCGCGTCGGTTGC < SEQ ID NO.:12)-3' (25 nucleotides). BHQ is an abbreviation for Black Hole Quencher.

Microarray Surface (a Seventh) Probe ($S_2$ Probe):

5'-F-GCAACCGACGCGATACCGTTGAGGG (SEQ ID NO.:13) -3' (25 nucleotides)。 F is a fluorescent group such as FAM, Cy3.

Target substrate 2 (SEQ ID NO.: 14) :

5'-<u>TGTGTACTACACTGGCATCG</u>-**CATGGAGCACTGATATCGCCTTC**

**GAATAACAATTA**

   【    $\_T_{b2}-T_{a2}$   】【         **X₂ interval**

**TACCCGCGAGAAATCTTTGAG**-<u>CTAAGACTTACTGTCAGCCTGAGTAC</u>-3'

   】【      $T_{rev2}$      】

- a First primer, a second primer (10nM)
- a Fourth primer, a fifth primer (100nM)
- Quenching (a sixth) probe Q-$P_2$ (100nM)

The results of the experiment are shown in Figure 3. It can be observed from the experimental results that the reaction system allows independent, specific and quantitative detection of the double amplification product at $10^5$ and $10^3$ orders of magnitude copy input in feasibility experiments. In reactions 1 and 2, Ct = 7.1 for detection of target 1 and Ct = 7.2 for detection of target 2, and the Ct of the two detection targets is also relatively close. Because both targets are amplified and detected simultaneously in the same detection cassette, this example, combined with the validation of the single reaction in Example 1, demonstrates the operability and advancement of the method in the present invention for multiplex qPCR amplification.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

**Claims**

1. A surface probe-based quantitative PCR detection system, the detection system comprising:

   (a) a solid support, the solid support having n sub-detection zones on a main surface, wherein n is a positive integer of & 2 and at least one of the sub-detection zones is a surface quantification sub-detection zone; wherein each of the surface quantification sub-detection zone is independently immobilized with a microarray surface probe, the microarray surface probe being a single stranded nucleic acid, and one end of the microarray surface probe is immobilized on the surface of the solid support, and the microarray surface probe carries a first detectable marker, the first detectable marker being selected from the group consisting of: a fluorescent group, luminescent group, luminescent marker, quantum dot, and a combination thereof;
   (b) a primer pair specific to the sequence to be detected, comprising a first primer and a second primer;
   (c) a quenching probe, a quencher is attached to one side or one end or in the middle of the quenching probe;

   and wherein the quenching probe and at least one microarray surface probes of the surface quantification sub-detection zone can be combined to form a double-stranded structure, and in the double-stranded structure, the quenching group of the quenching probe causes the signal of the first detectable marker (e.g. fluorescent group) of the microarray surface probe to be quenched in whole or in part; when the concentration of the quenching probe in the detection system is reduced, the degree of quenching of the signal of the first detectable marker (e.g. fluorescent group) of the microarray surface probe of at least one surface quantification sub-detection zone is reduced.

2. The detection system of claim 1, wherein the quenched in part means the degree of quenching of the signal of the first detectable marker (e.g. fluorescent group) of the microarray surface probe after addition by & 50%, preferably, $\geq$ 70%, more preferably & 80%, compared to that before addition of the quenching probe.

3. The detection system of claim 1, the reduced degree of quenching means that as the concentration of the quenching probe in the detection system decreases, the degree of quenching of the signal of the first detectable marker (e.g. fluorescent group) of the microarray surface probe is $\leq$ 50%, preferably $\leq$ 30%, more preferably, $\leq$ 20%.

4. The detection system of claim 1, the first primer has a structure of Formula I:

$$5\text{'-}T_{ai}\text{'-}L_{ai}\text{-}C_a\text{-}L_{b\,i}\text{-}P_i\text{-}L_{c\,i}\text{-}T_{bi}\text{-}T_{ai}\text{-}3\text{'} \quad (I)$$

   wherein $i$ is the $i$ number of a multiplex (n') detection, $i$ is a positive integer and $1 \leq i \leq$ n'; n' is a positive integer and $1 \leq$ n' $\leq$ the number of a solid support sub-detection zone, preferably n' is 2-100, more preferably, n' is 3-20;
   $T_{ai}$ is the a-part of the forward-specific sequence of the $i$-th target gene.
   $T_{ai}$' is a reverse complementary sequence of $T_{ai}$.
   $T_{bi}$ is the b-part of the forward specific sequence of the $i$-th target gene; and $T_{ai}$, $T_{bi}$ are directly adjacent to each other and together form the forward specific sequence of the target gene;
   $P_i$ is the sequence of the marker probe (barcode index probe) that marks the amplification of the $i$ number of gene;
   $C_a$ is the sequence of the forward universal amplification primer;
   $L_{ai}$, $L_{bi}$, $L_{ci}$ each independently being none or a flexible transition region, the flexible transition region being selected from the group consisting of: a flexible transition nucleic acid fragment of 1-15 nt in length, a flexible transition polymer fragment of 1-10 nt in length, and a combination thereof;
   each "-" is independently a bond or a nucleotide linker sequence.

**5.** The detection system of claim 4, $T_{ai}$ has a length of 6-20bp, preferably, 8-16bp, more preferably, 9-12bp.

**6.** The detection system of claim 4, the length of $T_{bi}$ is 3-50bp, preferably, 5-22bp, more preferably, 10-15bp.

**7.** The detection system of claim 1, the second primer has a structure of Formula II:

$$5\text{'}-C_b-L_{di}-T_{revi}\text{'}-3\text{'} \quad (\text{II})$$

wherein

$C_b$ is a reverse universal amplification primer sequence;
$L_{di}$ is none or a flexible transition region, the flexible transition region being a flexible transition nucleic acid fragment of 1-10 nt in length;
$T_{revi}\text{'}$ is a specific sequence at the 3' end of the $i$ number targeting gene.

**8.** The detection system of claim 1, the quenching probe has a structure of Formula III:

$$5\text{'}-Q-P_i-3\text{'} \quad (\text{III})$$

wherein Q is a quenching group;
$P_i$ is a sequence of the marker probe (barcode index probe) that marks the amplification of the $i$ number of gene.

**9.** A method for performing a quantitative PCR detection comprising the steps of:

(a) providing a sample to be tested and a quantitative PCR detection system based on a surface probe of claim 1;
(b) performing PCR amplification of the sample to be tested with the quantitative PCR detection system under suitable conditions for PCR amplification.
(c) detecting the signal of a first detectable marker of one or more surface quantification sub-detection zones on a solid support during or after the PCR amplification; and
(d) analyzing the detected signal of the first detectable marker, thereby obtaining a quantitative detection result of the sample to be tested.

**10.** A kit for a quantitative PCR detection comprising:

(a) a first container and a solid support located in the first container ,

the solid support having n sub-detection zones on a main surface, wherein n is a positive integer of & 2 and at least one of the sub-detection zones is a surface quantification sub-detection zone;
wherein each of the surface quantification sub-detection zone is independently immobilized with a microarray surface probe, the microarray surface probe being a single stranded nucleic acid, and one end of the microarray surface probe is immobilized on the surface of the solid support, and the microarray surface probe carries a first detectable marker, the first detectable marker being selected from the group consisting of: a fluorescent group, luminescent group, luminescent marker, quantum dot, and a combination thereof;

(b) a second container and (b1) a primer pair specific to the sequence to be detected, comprising a first primer, a second primer; and (b2) a quenching probe, a quencher is attached to one side or one end or in the middle of the quenching probe, located in the second container;
and wherein the quenching probe and at least one microarray surface probes of the surface quantification sub-detection zone can be combined to form a double-stranded structure, and in the double-stranded structure, the quenching group of the quenching probe causes the signal of the first detectable marker (e.g. fluorescent group) of the microarray surface probe to be quenched in whole or in part; when the concentration of the quenching probe in the detection system is reduced, the degree of quenching of the signal of the first detectable marker (e.g. fluorescent group) of the microarray surface probe of at least one surface quantification sub-detection zone is reduced;
(c) optionally a third container and a buffer or buffer component for PCR amplification located in the third container;
(d) optionally a fourth container and a polymerase for PCR amplification located in the fourth container;

(e) optionally a fifth container and a universal amplification primer pair located in the fifth container; and

(f) optionally a specification, the specification describes a method for performing a quantitative PCR detection.

Figure 1

Figure 2

Figure 3

Figure 4

thermal energy

Light source

CCD

Figure    5

## INTERNATIONAL SEARCH REPORT

| International application No. |
|---|
| **PCT/CN2021/082743** |

**A.    CLASSIFICATION OF SUBJECT MATTER**

C12Q 1/6851(2018.01)i

According to International Patent Classification (IPC) or to both national classification and IPC

**B.    FIELDS SEARCHED**

Minimum documentation searched (classification system followed by classification symbols)

C12Q

Documentation searched other than minimum documentation to the extent that such documents are included in the fields searched

Electronic data base consulted during the international search (name of data base and, where practicable, search terms used)

CNABS, CNTXT, DWPI, SIPOABS, CATXT, USTXT, EPTXT, WOTXT, KRTXT, JPTXT, CPEA, CNKI, WEB OF SCIENCE, PUBMED, 百度学术搜索: 芯片, 微阵列, PCR, 固相, 探针, 淬灭, 猝灭, 荧光, 标记物, 引物, 深圳闪量科技有限公司, chip, microarray, solid, probe, quench, fluorescent, label, primer GenBank, EMBL, China Patents Biological Sequence Search System sequence search for SEQ ID NOs: 1-14

**C.    DOCUMENTS CONSIDERED TO BE RELEVANT**

| Category* | Citation of document, with indication, where appropriate, of the relevant passages | Relevant to claim No. |
|---|---|---|
| Y | CN 110016500 A (SHENZHEN SHANLIANG TECHNOLOGY CO., LTD.) 16 July 2019 (2019-07-16)<br>   see claims 1-10 | 1-10 |
| Y | CN 103975061 A (EIKEN CHEMICAL CO., LTD.) 06 August 2014 (2014-08-06)<br>   see claims 1-6 | 1-10 |
| A | US 2011105348 A1 (EVOTEC AG.) 05 May 2011 (2011-05-05)<br>   see entire document | 1-10 |
| PX | CN 111394432 A (SHENZHEN SHANLIANG TECHNOLOGY CO., LTD.) 10 July 2020 (2020-07-10)<br>   see claims 1-10 | 1-10 |

☐ Further documents are listed in the continuation of Box C.          ☑ See patent family annex.

| | |
|---|---|
| \*    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **24 May 2021** | **17 June 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/082743**

**Box No. I**     **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
Information on patent family members

International application No.

**PCT/CN2021/082743**

| Patent document cited in search report | | | Publication date (day/month/year) | Patent family member(s) | | | Publication date (day/month/year) |
|---|---|---|---|---|---|---|---|
| CN | 110016500 | A | 16 July 2019 | None | | | |
| CN | 103975061 | A | 06 August 2014 | EP | 2787077 | A4 | 01 July 2015 |
| | | | | AU | 2012333771 | B2 | 05 October 2017 |
| | | | | ES | 2755934 | T3 | 24 April 2020 |
| | | | | HK | 1199287 | A1 | 26 June 2015 |
| | | | | JP | 6085564 | B2 | 22 February 2017 |
| | | | | WO | 2013065574 | A1 | 10 May 2013 |
| | | | | EP | 2787077 | A1 | 08 October 2014 |
| | | | | AU | 2012333771 | A1 | 26 June 2014 |
| | | | | CA | 2857308 | A1 | 10 May 2013 |
| | | | | US | 2014349295 | A1 | 27 November 2014 |
| | | | | CA | 2857308 | C | 01 October 2019 |
| | | | | TW | I608103 | B | 11 December 2017 |
| | | | | HK | 1249143 | A1 | 26 October 2018 |
| | | | | EP | 2787077 | B1 | 23 October 2019 |
| | | | | KR | 20140091562 | A | 21 July 2014 |
| | | | | CN | 108103145 | A | 01 June 2018 |
| | | | | TW | 201333209 | A | 16 August 2013 |
| | | | | JP | WO2013065574 | A1 | 02 April 2015 |
| | | | | KR | 101958048 | B1 | 13 March 2019 |
| | | | | US | 10876160 | B2 | 29 December 2020 |
| US | 2011105348 | A1 | 05 May 2011 | US | 8580577 | B2 | 12 November 2013 |
| | | | | DE | 60313894 | D1 | 28 June 2007 |
| | | | | EP | 1431398 | A1 | 23 June 2004 |
| | | | | EP | 1585829 | B1 | 16 May 2007 |
| | | | | US | 2006188880 | A1 | 24 August 2006 |
| | | | | DE | 60313894 | T2 | 17 January 2008 |
| | | | | AU | 2003290106 | A1 | 14 July 2004 |
| | | | | AT | 362549 | T | 15 June 2007 |
| | | | | EP | 1585829 | A1 | 19 October 2005 |
| | | | | WO | 2004057023 | A1 | 08 July 2004 |
| CN | 111394432 | A | 10 July 2020 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAMBROOK et al.** Molecular Cloning: A Laboratory Manual. Cold Spring Harbor Laboratory Press, 1989